# EUROPÄISCHE PATENTSCHRIFT

(11) **EP 1 779 886 B1**
(45) Veröffentlichungstag und Bekanntmachung des Hinweises auf die Patenterteilung: **18.02.2009**
(21) Anmeldenummer: 06022213.0
(22) Anmeldetag: 24.10.2006
(51) Int. Cl.: A61M 15/06, A24F 47/00

(54) **Tascheninhalator**
Pocket inhaler
Inhalateur de poche

(30) Priorität: 27.10.2005 AT 17532005
(43) Veröffentlichungstag der Anmeldung: 02.05.2007
(73) Patentinhaber: Werner, Daniel Sherlock, 4400 Steyr (AT)
(72) Erfinder: Werner, Daniel Sherlock, 4400 Steyr (AT)

(56) Entgegenhaltungen:
- EP-A2- 0 277 519
- US-A- 2 342 853
- US-A- 5 692 291
- US-A- 5 730 158

## Beschreibung

Die Erfindung betrifft einen Tascheninhalator mit externer Beheizung zur Erzeugung von dampf- oder rauchförmigem, aroma- und/oder wirkstoffhaltigem Aerosol aus Pflanzeninaterial. Der Begriff "Aerosol" steht für die feinste Verteilung fester oder flüssiger Stoffe in Gas, zum Beispiel Rauch, Nebel oder Dampf. Die Erfindung kann sowohl zur Erzeugung von dampfförmigem als auch rauchförmigem Aerosol benutzt werden, wobei die Möglichkeit der Erzeugung von dampfförmigem Aerosol sowohl erfindungs- als auch anwendungstechnisch vorrangig ist. Aroma- und/oder Wirkstoffdampftherapien können bei unterschiedlichsten Erkrankungen (z.B. der Atemwege) sowie bei der Rauchentwöhnung tabaksüchtiger Menschen zur Anwendung kommen.

Ein simples Verfahren zur Erzeugung von Aerosol aus Pflanzenmaterial stellt dessen Erhitzung bei stehender Luft dar. Bei Erreichen der notwendigen Temperatur beginnt das Pflanzenmaterial die Dämpfe auszusondern. Diese können dann vorsichtig abgesaugt werden, wobei sowohl wegen der hohen Temperatur der Dämpfe als auch wegen ihrer hohen Konzentration Vorsicht vor einer Überreizung der Atemwege angebracht ist. Dieses Verfahren findet vorwiegend bei Tascheninhalatoren mit externer Beheizung Verwendung, die zugunsten der Größe und des Gewichts auf ein geeignetes Kühlsystem und eine geeignete Steuerung des Volumens des Heißluftstroms bzw. überhaupt auf den Heißluftstrom verzichten.

Dieser Art von Tascheninhalatoren ist ihr simpler Aufbau aber auch die damit verbundenen Probleme gemeinsam. In der Regel handelt es sich um einen zumeist gläsernen Behälter, beispielsweise in Glühbirnen- oder Rohrform, in den das Pflanzenmaterial gegeben wird. Auf dem Behälter wird dann ein kleines Mundstück oder Röhrchen so angebracht, dass der Inhalt des Behälters abgesaugt werden kann und zugleich durch eine kleine Öffnung Luft in denselben nachströmen kann. Zuerst - und hier beginnen die Probleme - wird der Boden des Behälters von außen - etwa mit einem herkömmlichen Feuerzeug - solange erhitzt, bis sich Dampf in dem Behälter zu sammeln beginnt. Da das Pflanzenmaterial direkt auf dem Boden des Behälters liegt, kann es schnell zu brennen beginnen. Hat man dies durch viel Erfahrung oder auch durch Glück zu verhindern gewusst, muss der Dampf vorsichtig abgesaugt und vor dem eigentlichen Inhalieren in der Mundhöhle zwischengelagert werden. Vorsicht ist aus den bereits erwähnten Gründen Dampfkonzentration- und Temperatur geboten: Es ist keine direkte Lungenbeförderung, also kein direktes Einatmen der Aroma- und Wirkstoffdämpfe ohne Reizung der Atemwege möglich. Abgesehen von der Reizung der Lungen bei unvorsichtigem Absaugen muss auch der Verdampfungsprozess selbst als mangelhaft eingestuft werden, da eine vollständige Ausbeute der Aroma- und/oder Wirkstoffe nicht gewährleistet ist. Letzteres hat vor allem damit zu tun, dass das Pflanzenmaterial für einen vollständigen Verdampfungsprozess auf einen kleinen Raum konzentriert sein sollte, und zwar weder zu lose noch zu fest gepresst. Dadurch findet die Verdampfung beim gesamten Pflanzenmaterial gleichzeitig und im selben Temperaturbereich statt. Da herkömmliche Tascheninhalatoren aufgrund der fehlenden Kühlung und Luftstromkontrolle auch bei der Wirkstoffaufhahme klare Nachteile gegenüber ausgereiften Heißluftstrominhalatoren aufweisen, sind sie für den medizinischen Einsatz schlecht geeignet.

Es existieren verschiedene Geräte, die Aroma- und/oder Wirkstoffdämpfe (dampfförmiges, aroma- und/oder wirkstoffhaltiges Aerosol) durch einen am Pflanzenmaterial vorbeigeführten Heißluftstrom erzeugen. Bei diesem Verfahren müssen die Pflanzen nicht erst aufwendig extrahiert werden, es sind keine Lösungsmittel im Spiel und es kommt auch nicht zur schadstofflastigen Verbrennung des Pflanzcnmaterials. Als ausgereifteste Lösung für einen auf dem Heißluftstromprinzip basierenden Inhalator gilt zur Zeit ein vulkanförmiges Gerät mit der Patentschrift DE 198 03 376 C1 (EP0933093A2, US 6,513,524 B1).

Grundsätzlich erlaubt es die Beschaffenheit der bestehenden Geräte, die entstehenden Dämpfe so einzuatmen, dass dies nicht zu Reizungen des Atemsystems führt. Die Dampfkonzentration wird einerseits durch ein geeignetes Heißluftvolumen gering gehalten, andererseits wird der entstehende Dampf durch lange Leitungswege, ein geeignetes Auffangsystem oder Ähnliches abgekühlt Dies bewirkt aber zugleich, dass die auf Heißluftstrom basierenden Inhalatoren nur schlecht für den außerhäuslichen Gebrauch geeignet sind, da sie aufgrund ihres Gewichtes und/oder ihrer Größe nicht bequem mitgenommen werden können. Ein weiteres Problem bei der außerhäuslichen Nutzung von auf dem Heißluftstromprinzip basierenden Inhalatoren stellt die Energiequelle dar.

Handelt es sich um elektrische Geräte, benötigt man eine Stromquelle, funktionieren die Geräte mit externer Beheizung, erfordern sie aufgrund ihrer Dimensionierung und Funktionsweise einen die Größe und Funktionalität eines herkömmlichen Feuerzeugs überschreitenden Energiespender. Andererseits existiert kein auf ein akzeptables Taschenformat reduziertes Heißluftstromdampfgerät, welches voll funktionstüchtig ist, schnell und ohne speziell angepasste Hilfsmittel beheizt werden kann und eine effiziente Kühlung des Aerosols garantiert. Ein weiteres Problemfeld bei Tascheninhalatoren ist das Fehlen von besonders angepassten Vorrichtungen für den Transport.

Es gab in der Vergangenheit mehrere Versuche, brauchbare Modelle von Tascheninhalatoren zu kreieren, wobei beabsichtigt wurde, diese sowohl optisch als auch funktionell so weit als möglich herkömmlichen Zigaretten anzupassen. Dieser Anspruch wirkte sich sowohl auf die Dimensionierung als auch auf die Ausführung aus. So etwa die Anmeldungen EP 0340808 A2 und EP 0305788 A 1 und die dem Anmeldungsgegenstand am nächsten kommende in der EP 0277519 A2 beschriebene zigarettenförmige Einrichtung zur Inhalation von Aerosolen. Allen gemeinsam ist die physische Trennung von aerosolbildendem Material und dem zur Erwärmung des Materials verwendeten brennbarem Material, welches in die Einrichtungen integriert ist. Weiters ist bei allen ein - gegebenenfalls auswechselbares - Mundstück vorgesehen, welches die entstehende Hitze von den Lippen des Benutzers fernhalten und für die Abkühlung des Aerosols sorgen soll.

Ein Mundstück ist allerdings primär aus hygienischen Gründen sinnvoll, seine kühlende Wirkung ist aber beschränkt und kann niemals ein ausgereiftes Kühlsystem ersetzen. Besonderen Wert legen die genannten Anmedlungen darauf, dass die gesamte ins Gerät eindringende Luft am aerosolbildenden Material vorbeigeführt wird und dass somit keine Frischluft direkt in Mundhöhle des Benutzers gelangen kann. Der scheinbare Vorteil stellt aber sowohl temperaturtechnisch als auch hinsichtlich der Lungenverträglichkeit des Aerosols einen eindeutigen Nachteil dar, denn bewusst beigemengte Frischluft kann die effiziente Kühlung des Aerosols und seine erleichterte Inhalation garantieren.

Bei den Anmeldungen EP 0340808 A2 und EP 0305788 A1 ist trotz der Trennung des brennbaren Heizmaterials und des aerosolbildenden Materials im Inneren des Inhalators nicht ausgeschlossen, dass aus der Verbrennung des Heizmaterials resultierende Rückstände in die Lungen des Benutzers gelangen. Die Anmeldung EP 0277519 A2 stellt diesbezüglich eine eindeutige Verbesserung dar, weil die zur Bildung des Aerosols notwendige Luft durch seitliche Öffnungen in das Innere des Geräts gelangt. Bei allen beschriebenen Ausführungsmöglichkeiten wird allerdings weiterhin auf die zentrale Bedeutung eines Dichtrings im oberen Bereich des Geräts hingewiesen, der das direkte Einströmen von Außenluft in die Mundhöhle verhindert. Die daraus resultierenden Nachteile wurden bereits geschildert. Die Gefahr der ungewollten Inhalation von Verbrennungsnebenprodukten der Hitzequelle wird durch die seitlichen Einstromöffnungen auf eine Minimum reduziert. An der Tatsache von sich im Raum verteilenden und je nach Material mehr oder weniger stark riechenden Verbrennungsnebenprodukten ändert das allerdings nichts, zumal die Dauer des Verbrennungsvorganges bewusst jener der Verbrennungsdauer einer herkömmlichen Zigarette angepasst ist. Die externe Hitzezufuhr ist in diesem Sinn nicht perfekt gelöst, sie könnte schneller und schadstoffarmer erfolgen. In keinem der erwähnten Anmeldungsgegenstände ist die Möglichkeit eines regulierbaren Füllraums vorgesehen, also die Möglichkeit, mehr oder weniger aerosolbildendes Material im Inneren des Geräts plazieren zu können, ohne es dabei zu stark zusammendrücken oder, im Gegenteil, zu lose lassen zu müssen. Die Dichte des Füllmaterials ist für die Qualität der Verdampfung allerdings entscheidend.

Der vorliegenden Erfindung liegt die Aufgabe zugrunde, einen Tascheninhalator der eingangs geschilderten Art so weiterzubilden, dass er die vorgenannten Nachteile möglichst überwindet und seine Vorteile mit der Funktionalität eines ausgereiften Heißluftstrommodells vereint.

Die Aufgabe wurde dadurch gelöst, dass ein beidseitig offenes inneres Rohr vorgesehen ist, das auf einer Seite trichterförmig erweitert ist und dessen Innendurchmesser auf der anderen Seite in eine nach außen führende Verengung übergeht und dass auf das beidseitig offene innere Rohr im Bereich des die Verengung aufweisenden Endes ein Hohlzylinder aufgesteckt ist, der mit einer oder mehreren axial verlaufenden inneren Längsrillen versehen ist, die in eine oder mehrere Durchbrechungen der Mantelwand übergehen, und dass über das trichterförmig erweiterte Ende des inneren Rohres ein weiteres einseitig offenes äußeres Rohr bis über einen Teil des aufgesteckten Hohlzylinders ohne vollständige Abdeckung seiner Durchbrechung(en) geführt und am Hohlzylinder befestigt ist.

Es kommt zu keinem direkten Kontakt zwischen der Hitzequelle und dem Pflanzenmaterial. Nach dem Erhitzen des geschlossenen Endes des einseitig offenen Rohres werden beim Saugen am Hohlzylinder oder am optionalen Mundstück des Inhalators getrennte und kontrollierte Heiß- und Kaltluftströme erzeugt. Die trichterförmige Erweiterung des beidseitig offenen Rohres erleichtert sowohl das Füllen des Inhalators als auch die Stabilisierung des Rohres, die Verkleinerung des Innendurchmessers bremst den Heißluftstrom und verhindert sein überhastetes Eintreten in die Mundhöhle. Das durch den Heißluftstrom erzeugte Aerosol wird vor dem Eintritt in die Mundhöhle durch den Kaltluftstrom, also die bewusste und gesteuerte Beimengung von Frischluft, abgekühlt. Zusätzlich sorgt der Kaltluftstrom auch deshalb für eine angenehmere Inhalation, weil er das Gesamtvolumen der eingeatmeten Aerosol-Luft-Mischung erhöht.

Des weiteren ist erfindungsgemäß vorgesehen, dass die Wand des Hohlzylinders eine konzentrische, ringförmige Erweiterung aufweist, die sich zumindest teilweise-im Bereich der Durchbrechung(en) befindet und deren Außendurchmesser größer ist als der Innendurchmesser des einseitig offenen äußeren Rohres und auch größer als der Innendurchmesser eines optionalen aufsteckbaren Mundstücks, um als Anschlag für das aufgeschobene äußere Rohr und für das aufgesteckte Mundstück zu fungieren.

Dadurch wird sichergestellt, dass das einseitig offene äußere Rohr nicht über die Durchbrechung(en) der Mantelwand des Hohlzylinders geschoben werden kann und somit die in das Gerät eindringende Luft nicht behindert wird. Bei Verwendung des optionalen Mundstücks wird derselbe Effekt erzielt, die Durchbrechung bleibt frei.

Der erfindungsgemäße Tascheninhalator ist weiters durch ein abnehmbares. Gitter gekennzeichnet, das auf die erweiterte Seite des beidseitig offenen inneren Rohres gesteckt ist.

Durch das Abnehmen des Gitters ist es möglich, das Pflanzenmaterial im Inneren des beidseitig offenen inneren Rohres zu platzieren. Durch neuerliches Anbringen des Gitters wird erstens verhindert, dass das Pflanzenmaterial aus dem beidseitig offenen inneren Rohr herausfallen kann, wodurch sein direkter Kontakt mit dem über das beidseitig offene innere Rohr geschobenen einseitig offenen äußeren Rohr vermieden wird. Zudem ist das Gitter so dimensioniert, dass sein Außcndürchmesser nur knapp unter dem Innendurchmesser des einseitig offenen äußeren Rohres liegt. Dadurch wird das beidseitig offene innere Rohr auf der Längsachse des einseitig offenen äußeren Rohres stabilisiert. Dies schützt das Pflanzenmaterial vor ungewollter Verbrennung und sorgt für eine gleichmäßige Aerosolbildung.

Ferner ist erfindungsgemäß ein verschiebbares Gitter im Inneren des beidseitig offenen inneren Rohres vorgesehen.

Das Verschieben dieses Gitters erlaubt die Adaptierung des Füllraums und der Fülldichte an die verwendete Menge Pflanzenmaterial. Der Füllraum ist der Platz zwischen dem abnehmbaren Gitter, das auf die erweiterte Seite des beidseitig offenen inneren Rohres gesteckt wird, und dem verschiebbaren Gitter im Inneren des beidseitig offenen inneren Rohres. Zusätzlich wirkt das verschiebbare Gitter als Filter.

Erfindungsgemäß ist weiters eine Transporthülse im Stiftdesign mit Stöpsel und Aufprallschutz vorgesehen. Das Design dieser besonders angepassten Vorrichtung für den Transport schützt die Benutzer vor indiskreten Fragen, die Hülse und der Stöpsel und Aufprallschutz schützen den Inhalator vor Beschädigungen.

### Figurenübersicht

Fig. 1 zeigt den in der Transporthülse im Stiftdesign integrierten Inhalator. Aus Gründen der Übersichtlichkeit werden die Umrisse der Transporthülse im Stiftdesign nur angedeutet

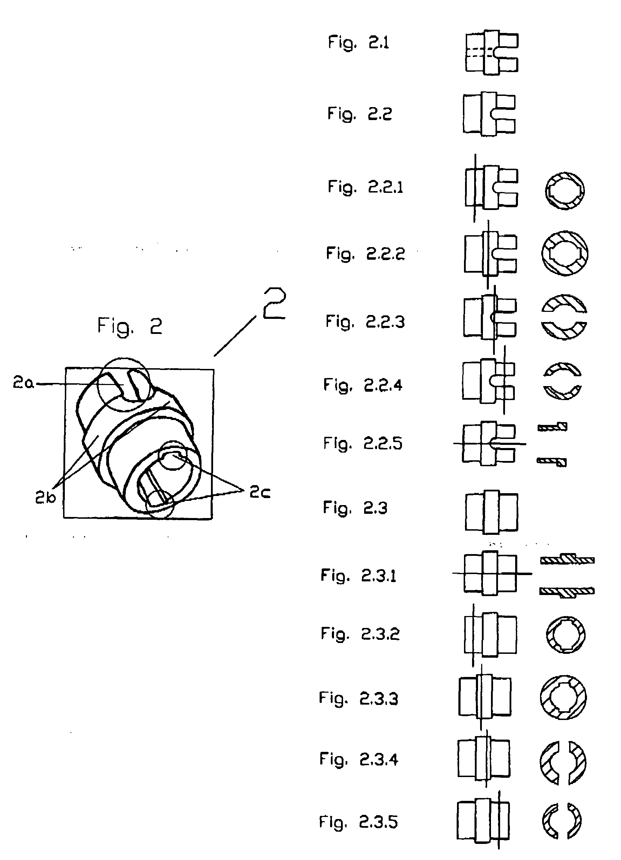

Fig. 2 zeigt perspektivisch eine mögliche Ausführungsform des Hohlzylinders 2 mit einer oder mehreren axial verlaufenden inneren Längsrillen, die in eine oder mehrere Durchbrechungen der Mantelwand übergehen. Der als 2a gekennzeichnete Bereich am oberen Rand der Darstellung verweist auf eine mögliche Ausführungsform der Durchbrechungen der Zylinderwand, 2b verweist auf die konzentrische, ringförmige Erweiterung der Zylinderwand, die sich zumindest teilweise im Bereich der seitlichen Durchbrechungen befindet, die als 2c gekennzeichneten Bereiche auf die axial verlaufenden Längsrillen im Innenraum des. Hohlzylinders.
   Fig. 2.1 zeigt eine Seitenansicht von Fig. 2 mit strichliert angedeuteten Tnnenstromkanälen
   Fig. 2.2 zeigt eine Seitenansicht von Fig. 2
      Fig. 2.2.1 zeigt einen Querschnitt durch Fig. 2.2 nach der Linie im Bereich des Aufsatzes für das optionale Mundstück 4
      Fig. 2.2.2 zeigt einen Querschnitt durch Fig. 2.2 nach der Linie in jenem Bereich der konzentrischen, ringförmigen Erweiterung 2b, wo diese keine seitlichen äußeren Durchbrechungen aufweist und daher durchgehend ist
      Fig. 2.2.3 zeigt einen Querschnitt durch Fig. 2.2 nach der Linie in jenem Bereich der konzentrischen, ringförmigen Erweiterung 2b, wo diese seitliche äußere Durchbrechungen aufweist und daher nicht durchgehend ist
      Fig. 2.2.4 zeigt einen Querschnitt durch Fig. 2.2 nach der Linie im Bereich des Aufsatzes für das einseitig offene äußere Rohr 5
      Fig. 2.2.5 zeigt einen Längsschnitt durch Fig. 2.2 nach der Linie im Bereich der inneren Längsrillen 2c und der seitlichen äußeren Durchbrechungen 2a
   Fig. 2.3 zeigt Fig. 2.2 um 90 Grad entlang der Längsachse gedreht
      Fig. 2.3.1 zeigt einen Längsschnitt durch Fig. 2.3 nach der Linie im Bereich ohne innere Längsrillen 2c und seitliche äußere Durchbrechungen 2a
      Fig. 2.3.2 zeigt einen Querschnitt durch Fig. 2.3 nach der Linie im Bereich des Aufsatzes für das optionale Mundstück 4
      Fig. 2.3.3 zeigt einen Querschnitt durch Fig. 2.3 nach der Linie in jenem Bereich der konzentrischen, ringförmigen Erweiterung 2b, wo diese keine seitlichen äußeren Durchbrechungen aufweist und daher durchgehend ist
      Fig. 2.3.4 zeigt einen Querschnitt durch Fig. 2.3 nach der Linie in jenem Bereich der konzentrischen, ringförmigen Erweiterung 2b, wo diese seitliche äußere Durchbrechungen aufweist und daher nicht durchgehend ist
      Fig. 2.3.5 zeigt einen Querschnitt durch Fig. 2.3 nach der Linie im Bereich des Aufsatzes für das einseitig offene äußere Rohr 5

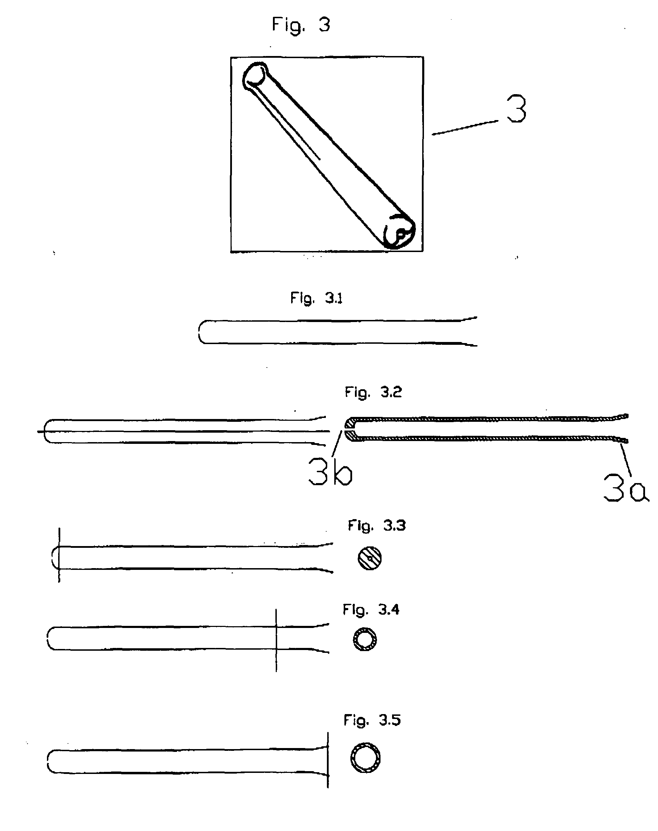

Fig. 3 zeigt perspektivisch eine mögliche Ausführungsform des beidseitig offenen inneren Rohres 3, das auf einer Seite trichterförmig erweitert ist 3a und dessen Innendurchmesser auf der anderen Seite in eine in eine nach außen führende Verengung 3b übergeht
   Fig. 3.1 zeigt eine Seitenansicht des beidseitig offenen inneren Rohres 3
   Fig. 3.2 zeigt einen Längsschnitt nach der Linie durch Fig. 3.1
   Fig. 3.3 zeigt einen Querschnitt durch Fig. 3.1 nach der Linie im Bereich der nach außen führenden Verengung 3b
   Fig. 3.4 zeigt einen Querschnitt durch Fig. 3.1 nach der Linie im mittleren Rohrbereich
   Fig. 3.5 zeigt einen Querschnitt durch Fig. 3.1 nach der Linie im trichterförmig erweiterten Bereich 3a

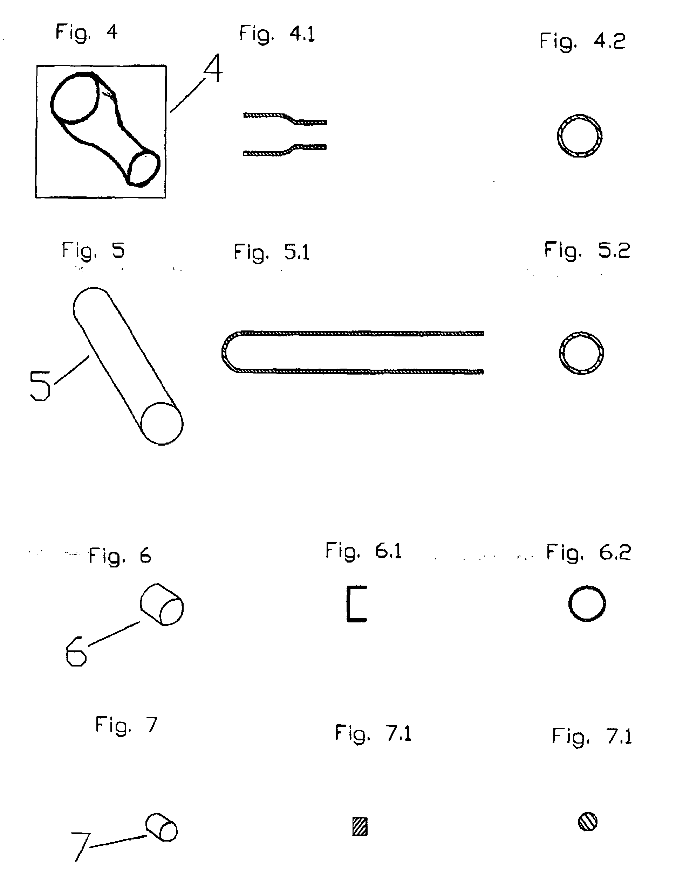

Fig. 4 zeigt perspektivisch eine mögliche Ausführungsform des optionalen Mundstücks 4
   Fig. 4.1 zeigt einen Längsschnitt durch Fig. 4
   Fig. 4.2 zeigt einen Querschnitt durch Fig. 4 im Bereich der Verbindung mit dem Hohlzylinder 2
Fig. 5 zeigt perspektivisch eine mögliche Ausführungsform des einseitig offenen Rohres 5
   Fig. 5.1 zeigt einen Längsschnitt durch Fig. 5
   Fig. 5.2 zeigt einen Querschnitt durch Fig. 5 im Bereich der Verbindung mit dem Hohlzylinder 2
Fig. 6 zeigt perspektivisch eine mögliche Ausführungsform des abnehmbaren Gitters 6
   Fig. 6.1 zeigt einen Längsschnitt durch Fig. 6
   Fig. 6.2 zeigt einen Querschnitt durch Fig. 6
Fig. 7 zeigt perspektivisch eine mögliche Ausführungsform des verschiebbaren Gitters 7
   Fig. 7.1 zeigt einen Längsschnitt durch Fig. 7
   Fig. 7.2 zeigt einen Querschnitt durch Fig. 7

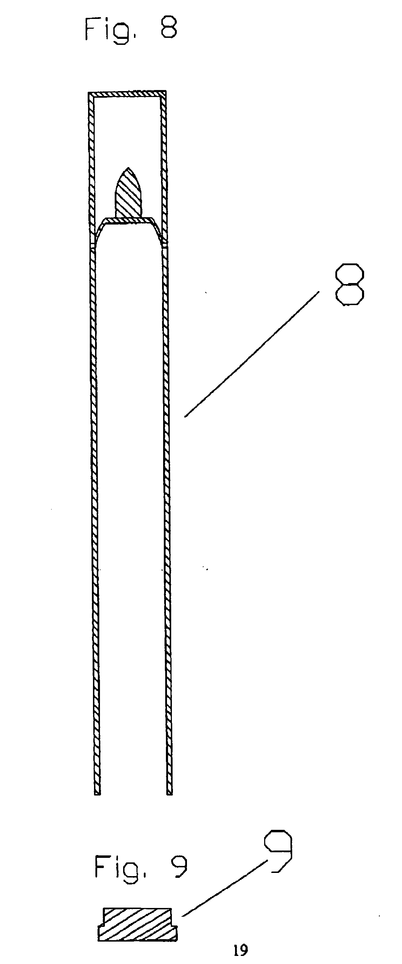

Fig. 8 zeigt einen Längsschnitt durch eine beispielsweise Ausführungsform der Transporthülse 8 im Stiftdesign
Fig. 9 zeigt einen Längsschnitt durch eine beispielsweise Ausführungsform des Stöpsels und Aufprallsehutzs 9 der Transporthülse 8 im Stiftdesign

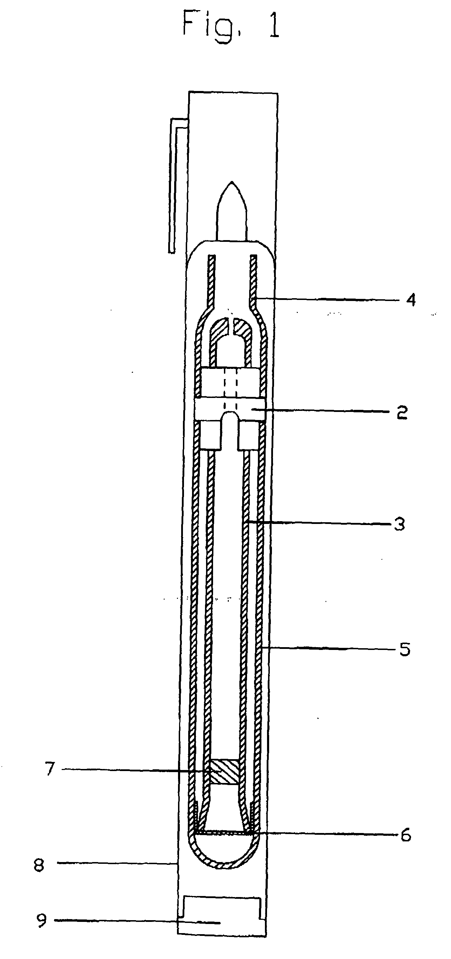

Die einzelnen Teile des Tascheninhalators müssen wie folgt zusammengesetzt werden (vgl. dazu auch Fig. 1): Der vorzugsweise aus Silikonkautschuk bestehende Hohlzylinder 2 mit einer oder mehreren axial verlaufenden inneren Längsrillen 2c, die in eine oder mehrere Durchbrechungen 2a der Mantelwand übergehen, wird im Bereich der nach außen führenden Verengung 3b auf das vorzugsweise aus hitzeresistentem Glas bestehende beidseitig offene innere Rohr 3 gesteckt. Der Innendurchmesser des Hohlzylinders 2 entspricht dem Außendurchmesser des beidseitig offenen inneren Rohres 3. Das vorzugsweise aus einem Metallsieb bestehende verschiebbare Gitter 7 wird auf der trichterförmig erweiterten Seite 3a in das beidseitig offene Rohr 3 geschoben und danach das beidseitig offene innere Rohr 3 ebenfalls von der trichterförmig erweiterten Seite 3a her mit dem zerkleinerten Pflanzenmaterial gefüllt. Nun wird das vorzugsweise aus einem Metallsieb bestehende abnehmbare Gitter 6 auf die trichterförmig erweiterte Seite 3a aufgesetzt. Der Füllraum für das Pflanzenmaterial liegt dementsprechend im Inneren des beidseitig offenen inneren Rohres 3 zwischen dem abnehmbaren Gitter 6 und dem verschiebbaren Gitter 7. Anschließend wird das vorzugsweise aus hitzeresistentem Glas bestehende einseitig offene äußere Rohr 5 über das abnehmbare Gitter 6 und das beidseitig offenen innere Rohr 3 geschoben bis es an der konzentrischen, ringförmigen Erweiterung 2b des aufgesteckten Hohlzylinders 2, die sich zumindest teilweise im Bereich der seitlichen Durchbrechungen 2a befindet anschlägt. Abschließend wird das vorzugsweise aus Glas bestehende optionale Mundstück 4 auf den dafür vorgesehenen Aufsatz (vgl. Fig. 2.2.1 und Fig. 2.3.2) des aufgesteckten Hohlzylinders 2 geschoben, bis es bei der konzentrischen, ringförmigen Erweiterung 2b des Hohlzylinders anschlägt.

Funktionsweise: Das geschlossene Ende des einseitig offenen äußeren Rohres 5 wird beispielsweise mit einer Flamme erhitzt. Durch Saugen am Hohlzylinder 2 oder am optionalen Mundstück 4 des betriebsbereiten Inhalators tritt über die auf die Längsrille(n) 2c stoßende(n) seitliche(n) äußere(n) Durchbrechung(en) 2a des Hohlzylinders 2 Luft in den Inhalator ein. Der eindringende Luftstrom kann grundsätzlich auf zwei verschiedenen Wegen in die Mundhöhle gelangen:

Der erste Weg ist jener des Heißluftstroms und verläuft zunächst einmal von den Durchbrechungen 2a für die Luftzufuhr zwischen dem einseitig offenen äußeren Rohr 5 und dem beidseitig offenen inneren Rohr 3 vorbei am abnehmbaren Gitter 6. Der Luftstrom ist dann am geschlossenen Ende des einseitig offenen Rohres 5 angelangt und wird an dieser Stelle indirekt durch das erhitzte einseitig offene Rohr 5 aufgeheizt. Nun setzt er seinen Weg durch das abnehmbare Gitter 6 in das Innere des beidseitig offenen inneren Rohres 3 fort. Er trifft dort zuerst auf das Pflanzettmaterial im Füllraum und bildet durch die Hitzeeinwirkung Aerosol. Vorbei am verschiebbaren Gitter 7 setzt der Luftstrom seinen Weg bis ans andere Ende des beidseitig offenen inneren Rohres 3 fort, wo durch die nach außen führende Verengung 3b des Innendurchmessers seine Geschwindigkeit und sein Volumen reduziert werden. Nach dem Austritt aus dem beidseitig offenen inneren Rohr 3 strömt die Luft entweder direkt oder über das optionale Mundstück 4 in die Mundhöhle.

Der zweite Weg, den die durch die auf die Längsrille stoßende seitliche äußere Durchbrechung 2a des Hohlzylinders 2 eindringende Luft einschlagen kann ist jener des Kaltluftstroms. Er führt durch die Längsrille 2c entlang des beidseitig offenen inneren Rohres 3 direkt zur Mundhöhle oder zum optionalen Mundstück 4. Beim Austritt aus der Längsrille 2c des Hohlzylinders 2 vermengt sich der Kaltluftstrom mit dem aus der nach außen führende Verengung 3b des beidseitig offenen inneren Rohres 3 strömenden Heißluftstrom, kühlt diesen ab und reichert ihn mit zusätzlichem Sauerstoff an.

Das Gesamtvolumen der durch den Tascheninhalator strömenden Luft entspricht der Summe aus dem Volumen des Heißluftstroms und dem Volumen des Kaltluftstroms. Für das korrekte Funktionieren dieses auf Druckausgleich basierenden Heiß- und Kaltluftsystems ist die Dimensionierung der Längsrille 2c des Hohlzylinders 2 und der Verengung 3b des beidseitig offenen inneren Rohres 3 entscheidend, also ein ausgeglichenes Verhältnis ihrer Durchzugsvolumina. Die durch die auf die Längsrille stoßende seitliche äußere Durchbrechung 2a eindringende Luft wird immer den Weg des geringsten Widerstands nehmen: Bei einer überdimensionierten zum optionalen Mundstück 4 führenden Längsrille und einer im Innendurchmesser extrem stark reduzierten Verengung 3b wäre der Kaltluftstrom überdimensioniert und der Heißluftstrom fiele praktisch aus, bei einem überdimensionierten Innendurchmesser der Verengung 3b in Verbindung mit kaum durchlässigen zum optionalen Mundstück 4 führenden Längsrillen 2c wäre der Heißluftstrom dominant und überhitzt.

Die Anzahl und Form der Längsrillen 2c und der seitlichen Durchbrechungen 2a für die Luftzufuhr kann variieren, solange die zum optionalen Mundstück 4 führenden Längsrillen 2c klein genug dimensioniert sind, um genügend Stauwirkung für den Kaltluftstrom zu haben und somit aufgrund des Druckausgleichs der Heißluftstrom durch den Füllkörper und das beidseitig offene innere Rohr 3 gesichert ist bzw. solange auch die Verengung 3b des Innendurchmessers des beidseitig offenen inneren Rohres 3 ihrerseits so dimensioniert ist, dass aufgrund des Druckausgleichs der Kaltluftstrom durch die zum optionalen Mundstück 4 führende Längsrillen 2c gesichert ist. Der Kaltluftstrom sorgt auch deshalb für eine angenehmere Inhalation, weil er das Gesamtvolumen der eingeatmeten Aerosol-Luft-Mischung und ihren Sauerstoffanteil erhöht und die Inhalation somit einem normalen, unverkrampften Atemzug ähnelt. Durch die auf die Längsrille stoßende(n) seitliche(n) äußere(n) Durchbrechung(en) 2a des Hohlzylinders 2 darf keine Stauwirkung und Aternbehinderung versursacht werden und muss daher ein Durchzugsvolumen gestattet werden, das über der Summe aus Kaltluft- und Heißluftstromvolumen liegt.

Das optionale Mundstück 4 ist aus hygienischen Gründen vorteilhaft, der Hohlzylinders 2 mit einer oder mehreren axial verlaufenden inneren Längsrillen 2c, die in eine oder mehrere Durchbrechungen 2a der Mantelwand übergehen, ist aber auch ohne es voll funktionsfähig, das Mundstück 4 also kein erfindungswesentlicher Bestandteil.

Trotz der geringen Größe und des geringen Gewichts des Geräts handelt es sich um einen Heißluftstrominhalator mit integrierter Kühlfunktion und Saucrstoffzufuhr. Obwohl die Verdampfung nahe des Mund- und Rachenraums stattfindet, sind Verbrennungen durch heiße Dämpfe ausgeschlossen und wird sogar das direkte Einatmen in die Lungen ermöglicht. Der Benutzer kann am Inhalator wie an einer Saueastoffflasche saugen, das Aerosol wird vermengt mit kühlender und sauerstofflicfemder Außenluft direkt in die Lungen befördert, ohne es zuerst in der Mundhöhle "zwischenlagern" zu müssen.

Der Verdampfungsprozess wird durch einen vorbeiziehenden Luftstrom ausgelöst und nicht über das Erhitzen der Pflanze bei stehender Luft und das darauf folgende Absaugen der entstandenen Dämpfe. Das System verhindert auch das Eindringen von Rußpartikeln und/oder anderen Schadstoffen der Flamme, da der Heißluftstrom erst am geschlossenen Ende und innerhalb des einseitig offenen Rohres 5 indirekt erhitzt wird. Der Inhalator kann grundsätzlich mit einer Vielzahl externer Hitzequellen betrieben werden.

Obwohl das bewusste Erhitzen des Inhalators bis zum Brennpunkt des Pflanzenmaterials möglich ist, um rauchförmiges Aerosol herzustellen, ist ein ungewolltes Entzünden des Pflanzenmaterials so gut wie ausgeschlossen. Direkt erhitzt wird ja nur das einseitig offene Rohr 5, das Pflanzenmaterial befindet sich allerdings im Füllraum im Inneren des beidseitig offenen inneren Rohres 3, zwischen dem abnehmbaren Gitter 6 und dem verschiebbaren Gitter 7. Das beidseitig offene innere Rohr 3 wird auf der Längsachse des einseitig offenen Rohres 5 an einem Ende durch den Hohlzylinder 2 und am anderen Ende durch die trichterförmige Erweiterung 3a des beidseitig offenen inneren Rohres 3 und das abnehmbare Gitter 6 zentriert und stabilisiert und liegt nirgends direkt am einseitig offenen Rohr 5 an. Das abnehmbare Gitter 6 verhindert den direkten Kontakt zwischen dem beidseitig offenen inneren Rohr 3, und dem einseitig offenen Rohr 5 und unterbindet das Herausfallen des Pflanzenmaterials aus dem Füllraum. Es ist also ausgeschlossen, dass Pflanzenmaterial in direkten Kontakt mit dem einseitig offenen Rohr 5 kommt und deshalb verbrennt. Da es gleichsam schwebend auf der Längsachse des direkt erhitzten einseitig offenen Rohres 5 positioniert ist, ist die Aufheizzeit zum Erreichen der für die Verdampfung notwendigen Mindesttemperatur lang genug, um ungewollte Überhitzung zu vermeiden. Sobald der Heißluftstrom Aroma- und/oder Wirkstoffdämpfe zu transportieren beginnt, kann dies der Benutzer geschmacklich wahrnehmen und die Flamme vom geschlossenen Ende des einseitig offenen Rohrs 5 entfernen. Ab dem ersten Wahrnehmen des Geschmacks kann beispielsweise eine herkömmliche Feuerzeugflamme noch etwa 10 Sekunden weiterheizen, ohne dass es zur Verbrennung kommt. Der Benutzer benötigt also kein Zeitmessgerät und kann den Inhalator ohne Hast betreiben. Unnötiger Aroma- und/oder Wirkstoffverlust wird dadurch vermieden, dass die Aroma- und/oder Wirkstoffe nur beim Einatmen durch den Inhalator in die Lungen befördert werden. Wird nicht am Gerät gesaugt, können sie nicht entweichen.

Das verschiebbare Gitter 7 ermöglicht eine Adaptierung des Füllraums und der Fülldichte an die verwendete Menge Pflanzenmaterial und erfüllt zusätzlich die Funktion eines Teilchenfilters, Durch Verschieben des Gitters 7 im Inneren des beidseitig offenen inneren Rohres 3 können unterschiedliche Mengen an Pflanzenmaterial in der jeweils idealen Dichte und auf einen kleinen Bereich beschränkt inhaliert werden. Da das verschiebbare Gitter 7 nur über leichten Druck an die Innenwand des beidseitig offenen Rohres 3 in seiner jeweiligen Position stabilisiert wird, kann es zum Beispiel mit einem Stück Draht von beiden Seiten des beidseitig offenen inneren Rohres 3 erreicht und in die gewünschte Richtung verschoben werden. Ein vollständiger und gleichmäßiger Verdampfungsprozess ist somit trotz variierender Füllmengen garantiert. Für die Reinigung kann das verschiebbare Gitter 7 zur Gänze aus dem beidseitig offenen inneren Rohr 3 geschoben werden.

Der Inhalator verfügt zudem über eine besonders angepassten Vorrichtung für den Transport. Die Transporthülse im Stiftdesign 8 schützt nicht nur das Gerät, sondern kann kranken Menschen auch unangenehme Fragen über die Gründe für die Verwendung eines Inhalators ersparen. Es handelt sich daher nicht um eine beliebige Plastikhülse, sondern um eine bewusst vom eigentlichen Inhalt ablenkende Verpackungstechnik. Man kann den Tascheninhalator ebenso wie einen Stift immer mit sich führen. Gedacht wurde dabei an herkömmliche Tascheninhalatoren oder aber auch an diverse sich im Umlauf befindende Asthmasprays, die wegen einer mangelhaften Verpackung indirekt Auskunft über das Leiden der Benutzer geben. Die Transporthülse im Stiftdesign 8 schafft- diesem Problem Abhilfe und ist in Verbindung mit dem Stöpsel und Aufprallschutz 9 so konstruiert, dass der Tascheninhalator beim unabsichtlichen Fallenlassen nicht zerberstet.

Im Einzelnen läuft die Inhalation so ab: Eine geeignete Pflanze auswählen und trocknen. Empfehlenswert sind beispielsweise Eukalyptus (Eucalyptus globulus Labill) oder Salbei (Salvia oficinalis). Diese Pflanzen können getrocknet und hygienisch verpackt beim Apotheker besorgt werden. Der Inhalator kann auch mit Tabak gefüllt und zur Rauchentwöhnung benutzt werden. Die Pflanze sorgfältig zerkleinern, weil dadurch die Oberfläche vergrößert wird. Das Zerkleinern der Pflanze ist bei allen bekannten Inhalatoren zur Erzeugung von aroma- und/oder wirkstoffhaltigen Dämpfen aus Pflanzenmaterial ausschlaggebend für die Qualität des Verdampfungsprozesses.

Die Transporthülse im Stiftdesign 8 öffnen und den Inhalator vorsichtig herausziehen. Nun den Inhalator selbst öffnen, indem das einseitig offene Rohr 5 vorsichtig vom Hohlzylinder 2 gezogen wird. Es empfiehlt sich, mit Zeigefinger und Daumen der einen Hand die konzentrische, ringförmige Erweiterung 2b des Hohlzylinders 2 festzuhalten, und mit den selben Fingern der anderen Hand das einseitig offene Rohr 5 gefühlvoll vom Hohlzylinder 2 herunterzuziehen. Das abnehmbare Gitter 6 vom beidseitig offenen inneren Rohr 3 ziehen und gegebenenfalls durch Verschieben des verschiebbaren Gitters 7 die Größe des Füllraums festlegen. Sodann wird der Füllraum bis oben mit der zerkleinerten getrockneten Pflanze gefüllt, ohne allerdings zu fest hineingepresst zu werden, denn dies behindert die Luftströme im Gerät und wirkt sich somit negativ auf den Verdampfungsprozess aus. Wird der Heißluftstrom zu sehr behindert, kann die Füllung nicht mehr gleichmäßig erhitzt werden, und es besteht die Gefahr einer Überhitzung im unteren Bereich. Für größere Füllmengen die Pflanze nicht gewaltsam in den Füllkörper pressen, sondern entweder den Füllraum vergrößern oder den Verdampfungsvorgang mehrmals mit kleineren Mengen wiederholen, da so die beste Dampfqualität erzeugt wird.

Nach dem Füllen das abnehmbare Gitter 6 wieder auf das beidseitig offene innere Rohr 3 und das einseitig offene Rohr 5 wieder auf den Hohlzylinder 2 stecken. Sicherstellen, dass der Füllraum zwischen dem abnehmbaren Gitter 6 und dem verschiebbaren Gitter 7 im vordersten Teil des einseitig offenen Rohres 5 ist Sollte dem nicht so sein, kann die Position des beidseitig offenes inneren Rohres 3 durch Verschieben des Hohlzylinders 2 angepasst werden. Das abnehmbare Gitter 6 sollte möglichst nahe beim geschlossenen Ende des einseitig offenen Rohres 5 sein, da sonst die Aufheizzeit unnötig verlängert wird. Bei Verwendung eines herkömmlichen Feuerzeugs beginnt man mit einer etwa 10 sekündigen Vorwärmphase. Den Inhalator am oberen Teil des einseitig offenen Rohres 5 anfassen. Die Hitze wandert nur langsam nach oben, und solange das Gerät nicht in unmittelbarer Nähe des erhitzten geschlossenen Endes des einseitig offenen Rohres 5 berührt wird, besteht keine Verbrennungsgefahr. Den Füllraum in den blauen bzw. unteren Teil der Flamme halten, wodurch die Flamme erstens weniger rußt und zweitens den gesamten Bereich gleichmäßiger erwärmt. Je stärker bzw. heißer die Flamme ist, desto kürzer ist auch die Vorwärmphase.

Das optionale Mundstück 4 - oder, wenn nicht vorhanden, den Hohlzylinder 2 direkt - fest mit den Lippen umschließen und den Inhalator gut halten. Nun einatmen und gegebenenfalls gleichzeitig weitererhitzen. Die Lippen so um das optionale Mundstück 4 legen, dass keine andere Luft als jene, die durch den Inhalator geht, in die Mundhöhle strömt. Der durch die seitlichen Durchbrechungen 2a des Hohlzylinders 2 eindringende Luftstrom ist leise zu hören. Der Vorgang erinnert an das Gefühl, durch eine Sauerstoffflasche einzuatmen. Man verspürt zwar einen leichten Widerstand, der Hohlzylinder 2 mit einer oder mehreren axial verlaufenden inneren Längsrillen 2c, die in eine oder mehrere Durchbrechungen 2a der Mantelwand übergehen, garantiert aber die Zufuhr zusätzlicher und kühlender Luft von außen. Es empfiehlt sich ein langsamer und langer Atemzug. Das Eindringen von Aerosol in den Rachenraum beginnt fast unverzüglich und wird mit zunehmender Erwärmung vor allem geschmacklich wahrgenommen. Selbst Nichtraucher empfinden dieses Gefühl nicht als störend, und müssen trotz des direkten Transports des Aerosols in die Lungen nicht husten. Die eingeatmete Mischung aus Luft und Aerosol ist beim Ausatmen optisch wahrnehmbar, wenn auch nicht immer sehr intensiv. Es kann durchaus vorkommen, dass man bei einer Verdampfung im unteren Temperaturbereich das Aerosol lediglich schmeckt, aber kaum bis gar nicht sieht. Der Vorgang kann in diesem Fall mit leicht erhöhter Hitzezufuhr wiederholt werden. War die Dampf Lun-Mischung gut sichtbar, dann reicht die gespeicherte Temperatur für einen direkt an den ersten anschließenden zweiten Zug ohne Hitzezufuhr von außen. Üblicherweise kann der Vorgang Vorheizen - Zug-2. Zug ohne Flamme mehrmals wiederholt werden.

Die genannten Intervalle gelten für Innenräume und können sich bei niedriger Außentemperatur oder windigen Verhältnissen verlängern. Sie können sich aber auch in Abhängigkeit von der Flamme, ihrer Größe und der durch sie erzeugten Hitze verkürzen. Grundsätzlich gilt: Möchte man dampfförmiges Aerosol, dann lieber zu wenig Hitze - das kann ja durch Nachheizen schnell gut gemacht werden - als zu viel. Ist rauchförmiges Aerosol gewünscht, dann bewusst bis zum Brennpunkt aufheizen. Es kommt zur Verbrennung der Pflanze, allerdings im untersten Temperaturbereich, weil das Volumen der für die Verbrennung benötigten Luft auf ein Minimum reduziert wird. Es ist nicht möglich, mit dem Gerät hastig und heiß zu rauchen, denn das beidseitig offene innere Rohr 3 und der Hohlzylinder 2 erfüllen auch bei der Verbrennung ihre Funktion. Das rauchförmige Aerosol wird von der nach außen führenden Verengung 3b des beidseitig offenen inneren Rohres 3 gebremst und noch vor dem Eintritt in die Mundhöhle zusätzlich abgekühlt und mit Sauerstoff von außen gespeist. Dies kann besonders für Tabakrauchsüchtige am Beginn der Entwöhnung eine Erleichterung sein oder aber auch ganz bewusst für "gesünderes" Rauchen benutzt werden. Beim Konsum von dampfförmigem Aerosol bleibt am Ende ein vollkommen ausgetrockneter aber nicht verbrannter Rest der Pflanze übrig. Einer der großen Vorteile des Verdampfens ist die hohe Ausbeute, eine sehr kleine Menge an Ausgangsmaterial, die bei Verbrennung vielleicht für einen Zug reichen würde, reicht im Inhalator für mehrere tiefe Züge aus, bei denen so gut wie nichts an Wirk- und/oder Aromastoffen verloren geht.

## Patentansprüche

1. Tascheninhalator mit externer Beheizung zur Erzeugung von dampf- oder rauchförmigem, aroma- und/oder wirkstoffhaltigem Aerosol aus Pflanzenmaterial, wobei in dem Tascheninhalator ein beidseitig offenes inneres Rohr (3) vorgesehen ist, dessen Innendurchmesser auf einer Seite in eine nach außen führende Verengung (3b) übergeht, und über die andere Seite (3a) des inneren Rohres ein weiteres einseitig offenes äußeres Rohr (5) geführt ist, wobei das geschlossene Ende des äußeren Rohres im Gebrauch mit einer externen Hitzequelle beheizbar list, **dadurch gekennzeichnet, dass** das Rohr (3) auf der anderen Seite (3a) trichterförmig erweitert ist und dass auf das beidseitig offene innere Rohr (3) im Bereich des die Verengung (3b) aufweisenden Endes ein Hohlzylinder (2) koaxial aufgesteckt ist, der mit einer oder mehreren axial verlaufenden inneren Längsrillen (2c) versehen ist, die in eine oder mehrere Durchbrechungen (2a) der Mantelwand übergeben, und dass das äußere Rohr (5) bis über einen Teil des aufgesteckten Hohlzylinders (2) ohne vollständige Abdeckung seiner Durchbrechung(en) (2a) geführt und am Hohlzylinder befestigt ist, wobei bei Verwendung die Luft durch das Erhitzen des geschlossenen Endes des einseitig offenen äußeren Rohres (5) und Saugen am Hohlzylinder (2) oder am optionalen Mundstück (4) über die Durchbrechungen (2a) in das Gerät eintritt, teilweise am erhitzten Teil des einseitig offenen äußeren Rohres (5) in das trichterförmig erweiterte Ende (3a) des beidseitig offenen inneren Rohrs (3) vorbeiströmt, indirekt aufgeheizt wird und über die Verengung (3b) und das optionale Mundstück (4) das Gerät verlässt, die eindringende Luft weiter teilweise über die Durchbrechungen (2a) direkt durch die axial verlaufenden Längsrillen (2c) in das optionale Mundstück (4) strömt, sich mit dem indirekt erhitzten Luftstrom vermengt und diesen noch vor dem Austritt aus dem Gerät abkühlt

2. Tascheninhalator nach Anspruch 1, **gekennzeichnet dadurch dass** die Wand des Hohlzylinders (2) eine konzentrische, ringförmige Erweiterung (2b) aufweist, die sich zumindest teilweise im Bereich der Durchbrechung(en) (2a) befindet und deren Außendurchmesser größer ist als der Innendurchmesser des einseitig offenen äußeren Rohres (5) und auch größer als der Innendurchmesser eines optionalen aufsteckbaren Mundstücks (4), um als Anschlag für das aufgeschobene äußere Rohr (5) und für das aufgesteckte Mundstück (4) zu fungieren.

3. Taseheninhalator nach Anspruch 1-2, **gekennzeichnet durch** ein abnehmbares Gitter (6), das auf die erweiterte Seite (3a) des beidseitig offenen inneren Rohres (3) gesteckt ist.

4. Tascheninhalator nach Anspruch 1-3, **gekennzeichnet dadurch dass** im Inneren des beidseitig offenen inneren Rohres (3) ein verschiebbares Gitter (7) vorgesehen ist.

5. Tascheninhalator nach Anspruch 1-4, **dadurch gekennzeichnet dass** eine Transporthülse im Stiftdesign (8) mit Stöpsel und Aufprallschutz (9) zum Schutz des Tascheninhalators vorgesehen ist.

## Claims

1. A pocket inhaler with external heating for the production of aerosol of vapor or smoke containing aromatic and/or active substances of plant material, being the pocket inhaler provided with an inner tube that is open on both ends (3) whose inner diameter leads into a narrow section heading outwards (3b), including an additional outer tube that is closed on one end (5) which is pushed over the other end (3a) of the inner tube, serving the closed end of the outer tube to be heated up with an external source of heat during usage, comprising a funnel-shaped opening on the other end (3a) of the tube (3) and further comprising a hollow cylinder (2) with one or more axially and lenghtways orientated inner slits (2c) that lead to one or more breaks (2a) in the casing, which is placed coaxially in the area of the narrow section (3b) of the inner tube that is open on both ends (3), in which the outer tube (5) is pushed partially over the placed hollow cylinder (2) and connected to it without covering completely its breaking(s) (2a), so that when used by heating the closed end of the outer tube (5) and sucking on the hollow cylinder (2) or the optional mouthpiece (4), air enters the device through the breaks (2a) and on one hand passes the heated part of the outer tube that is closed on one end (5), being heated up indirectly, and streaming into the funnel-shaped opening (3a) of the inner tube that is open on both ends (3) and coming out of the device through the narrow section (3b) and the optional mouthpiece (4) and on the other hand the air that enters the device through the breaks (2a) streams directly to the mouthpiece (4) through the inner slits (2c), mixing with the indirectly heated stream of air and cooling it before it comes out of the device.

2. A pocket inhaler according to claim 1, in which the casing of the hollow cylinder (2) shows a concentric, ring shaped widening (2b) which is at least partially placed in the area of the breaking(s) (2a) and whose outer diameter is bigger than the inner diameter of the outer tube that is closed on one end (5) and also bigger than the inner diameter of the optional mouthpiece (4) in order to function as a stop for the placed outer tube (5) and the placed mouthpiece (4).

3. A pocket inhaler according to the claims 1-2, in which a removable grid (6) is placed on the funnel-shaped opening (3a) of the inner tube that is open on both ends (3).

4. A pocket inhaler according to the claims 1-3, in which a movable grid (7) is placed inside the inner tube that is open on both ends (3).

5. A pocket inhaler according to the claims 1-4, in which a writer-shaped transport case (8) with a lid and crash protection (9) protects the pocket inhaler.

## Revendications

1. Inhalateur de poche à chaleur externe pour la production de l'aérosol de vapeur ou de fumée contenant des arômes et/ou des substances végétales où on a prévu dans l'inhalateur de poche un tube intérieur ouvert aux deux bouts (3) dont le diamètre intérieur mène à un bout à un rétrécissement qui conduit vers l'extérieur (3b) et où un autre tube ouvert à un bout (5) est conduit vers l'autre bout (3a) du tube intérieur et où pendant l'usage le bout fermé peut être chauffé par une source de chaleur externe, **caractérisé en ce que** le tube (3) à l'autre bout (3a) est élargi en forme d'entonnoir et **en ce que** sur le tube intérieur qui est ouvert aux deux bouts (3) est fixé dans le secteur du bout marqué par le rétrécissement (3b) un cylindre creux (2) de manière coaxiale qui dispose d'une ou de plusieurs rainures longitudinales intérieures (2c) longeant axialement et menant à une ou plusieurs perforations (2a) de la chemise du cylindre, et **en ce que** le tube extérieur (5) est conduit sur une partie du cylindre creux(2) sans couverture complète de ses perforations (2a) et est fixé sur le cylindre creux, puisque en cas d'application l'air - par le chauffage du bout fermé du tube extérieur ouvert à un seule bout (5) et en aspirant par le cylindre creux (2) ou par un embout buccal optionnel - entre à l'aide des perforations (2a) dans l'appareil, en partie en passant la partie chauffée du tube extérieur ouvert à un seul bout (5) il entre dans le bout élargi en forme d'entonnoir (3a) du tube ouvert aux deux bouts (3), se chauffe indirectement et ensuite il sort par le rétrécissement (3b) ou par l'embout buccal optionnel (4), et en partie l'air qui entre se répand par les perforations (2a) et directement par les rainures longitudinales axiales (2c) dans l'embout buccal optionnel (4) et ensuite il se mélange avec le courant d'air indirectement chauffé et il le refroidit encore avant son écoulement de l'appareil.

2. Inhalateur de poche selon la revendication 1 **caractérisé en ce que** la chemise du cylindre creux (2) dispose d'un élargissement concentrique et circulaire (2b) qui se trouve au moins en partie dans le secteur des perforations (2a) et dont le diamètre extérieur est plus grand que le diamètre intérieur du tube extérieur ouvert à un seul bout (5) et aussi plus grand que le diamètre intérieur de l'embout buccal optionnel (4) pour fonctionner comme seuil de résistance soit pour le tube extérieur (5) soit pour l'embout buccal optionnel (4) fixés.

3. Inhalateur de poche selon les revendications 1 - 2 **caractérisé en ce qu'**une grille qu'on peut enlever (6) est fixé sur la partie élargie (3a) du tube intérieur ouvert aux deux bouts (3).

4. Inhalateur de poche selon les revendications 1 - 3 **caractérisé en ce que** dans l'intérieur du tube intérieur ouvert aux deux bouts (3) est prévue une grille qu'on peut déplacer (7).

5. Inhalateur de poche selon les revendications 1 - 4 **caractérisé en ce que** l'appareil est protégé par une douille de transport en forme de crayon (8) avec un bouchon et une protection contre les chocs (9).
